**Europäisches Patentamt**

**European Patent Office**

**Office européen des brevets**

(11) Veröffentlichungsnummer: **0 311 556**
**A2**

# (12) EUROPÄISCHE PATENTANMELDUNG

(21) Anmeldenummer: 88730226.3

(22) Anmeldetag: 05.10.88

(51) Int. Cl.⁴: **A 61 B 17/58**

(30) Priorität: 06.10.87 DE 3734111

(43) Veröffentlichungstag der Anmeldung:
12.04.89 Patentblatt 89/15

(84) Benannte Vertragsstaaten:
AT BE CH DE ES FR GB GR IT LI NL SE

(71) Anmelder: **MECRON MEDIZINISCHE PRODUKTE GMBH**
**Nunsdorfer Ring 23-27**
**D-1000 Berlin 48 (DE)**

(72) Erfinder: **Kranz, Curt, Dipl.-Ing.**
**Kufsteiner Strasse 12**
**D-Berlin 62 (DE)**

**Sievers, Uve, Dr.**
**Ludwig-Guttmann-Strasse 13**
**D-6700 Ludwigshafen (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) Marknagel für die Behandlung von Knochenbrüchen nach dem Prinzip der Markraumnagelung und Marknagel-Werkzeug.

(57) Marknagel (1) für die Behandlung von Knochenbrüchen nach dem Prinzip der Markraumnagelung mit einem röhrenförmigen, vorzugsweise hohlzylindrischen Querschnitt, und einem sich zum Kopfende hin erweiternden bzw. im Nagelinneren verengenden Schaft oder abgeflachten Marknagelkopf (3). Ein Marknagel-Werkzeug zum Eintreiben, Extrahieren und zur Lageveränderung bei der Reposition mit einem verengten, hohlzylindrischen Marknagelkopf (3) weist mindestens zwei teilkreisförmige, gegeneinander verschwenkbare Zangenbacken (41, 42) auf mit nach innen bzw. außen gebogenen Enden zur Querschnittsverengung bzw. -erweiterung am Ende der Zangenbacken (41, 42).

Fig.1

EP 0 311 556 A2

## Beschreibung

## Marknagel für die Behandlung von Knochenbrüchen nach dem Prinzip der Markraumnagelung und Marknagel-Werkzeug

Die Erfindung betrifft einen Marknagel für die Behandlung von Knochenbrüchen nach dem Prinzip der Markraumnagelung.

Beispielsweise aus der Literaturstelle "Die Bündel-Nagelung" (Springer-Verlag, Berlin, (Göttingen, Heidelberg 1961, Seiten 3 bis 26) sind die Prinzipien der Marknagelung bzw. Bündel-Nagelung bekannt und es werden darin verschiedene Querschnittsformen der bei diesen Verfahren verwendeten Marknägel beschrieben. Neben den Nachteilen der mangelnden Biegungsstabilität und Drehstabilität der bekannten Marknägel wird insbesondere auf die zahlreichen, beim Entfernen der Marknägel nach dem Ausheilen des Bruches auftretenden Schwierigkeiten hingewiesen, die unter anderem zu Gelenkverletzungen und Infektionen führen und häufig ein Herausmeißeln des Nagels erforderlich machen.

Das gleiche trifft auf die Verwendung sogenannter Federnägel zu, deren kopfseitigen Enden zur späteren Entfernung des Federnagels abgebogen, aus der Einschlagstelle herausgeführt und zum Anliegen an die Einschlagstelle gebracht werden. Infolge ihrer abgebogenen Enden können die Federnägel jedoch infolge von Bewegungen des so geschienten Knochens zu Verletzungen an der Einschlagstelle führen.

Der Erfindung liegt die Aufgabe zugrunde, einen Marknagel zu schaffen, der bei hoher Biegesteifigkeit und Rotationsstabilität sowie geringer Materialermüdung neben einem einfachen Eintreiben des Marknagels und einer optimalen Stabilisierung der Fragmente sowie auch ein problemloses Positionieren und Extrahieren des Marknagels ermöglicht.

Diese Aufgabe wird mit den im kennzeichnenden Teil des Anspruchs 1 angegebenen Maßnahmen gelöst.

Mit der Erfindung wird eine Möglichkeit zur lokalen Krafteinleitung zum Extrahieren uund Positionieren bei Nägeln aus Kunststoffmaterial geschaffen, welche die im übrigen vorteilhaften Eigenschaften des Nagels oder Implantats nicht herabsetzt.

Bei der erfindungsgemäßen Lösung läßt sich zur lokalen Krafteinleitung in vorteilhafter Weise ein Werkzeug verwenden, welches das Kunststoffmaterial zur Führung des Nagels sicher erfaßt.

Durch eine Verjüngung oder Einschnürung im Krafteinleitungsbereich ist der Querschnitt der gesamten Anordnung trotz angesetztem Werkzeug nicht oder nur unwesentlich vergrößert, so daß die Bewegungsfreiheit beim Positionieren oder Ausziehen des Nagels erhalten bleibt. Auch trägt das den Kopf aufweisende Nagelende im implantierten Zustand nicht auf bzw. ist nicht durch das Gewebe hindurch ertastbar, wenn der Nagel voll im Knochen versenkt ist.

Eine vorteilhafte Ausgestaltung der erfindungsgemäßen Lösung ist dadurch gekennzeichnet, daß das Ende des Marknagelkopfes eine Verdickung - insbesondere im Anschluß an die Einschnürung - aufweist. Damit ist ein sicheres Ergreifen zum Extrahieren des Marknagels auch bei geringer Flächenpressung des Marknagelkopfes möglich.

In einer vorteilhaften Weiterbildung weist der Marknagel eine im Querschnitt hohlzylindrische Form und eine an der Spitze des Marknagels vorgesehene, scharf geschliffene Ringschneide auf.

In einer weiteren vorteilhaften Weiterbildung besteht der Marknagel aus einem aus Kohlenstoffasern gewirkten Schlauch und einem aushärtbaren Kunstharz. Der Schlauch kann im Kreuzverbund angeordnete Kohlenstoffasern aufweisen, während das aushärtbare Kunstharz aus einem bioverträglichen TEEC-Matrixmaterial bestehen kann, in das die Kohlenstoffasern eingebettet sind und das bei einer hohen Temperatur von vorzugsweise 380°C aushärtet. Die Form erfindungsgemäße Form des Nagelendes läßt sich insbondere bei faserverstärkten Kunststoffnägeln günstig herstellen.

In einer weiteren vorteilhaften Ausgestaltung ist der Marknagel C- oder J-förmig gebogen. Zusätzlich kann er ein in den Marknagelkopf eingesetztes, metallisches Krafteinleitungselement aufweisen, das beispielsweise aus einem Hohlzylinder bestehen kann, dessen Innenwand eine aufgerauhte, den Kraftschluß verbessernde, Oberfläche aufweist.

Eine vorteilhafte Ausgestaltung eines Marknagel-Werkzeuges zum Eintreiben, Extrahieren und zur Lageveränderung bei der Reposition für einen verengten, hohlzylindrischen Marknagelkopf bzw. einen abgeflachten Marknagelkopf ist durch mindestens zwei teilkreisförmige, gegeneinander verschwenkbare Zangenbacken mit nach innen bzw. außen gebogenen Enden versehen. Die Enden dieser Zangenbacken hinter greifen - jeweils nach innen bzw. außen gerichtet - die äußere Verdickung oder innere Querschnittserweiterung des Nagels im Bereich des Kopfendes. Ein Dorn bzw. eine in den Nagel eingeformte metallische Hülse bilden dabei ein Gegenstück zur Aufnahme des Anpreßdrucks. Dieser Anpreßdruck dient zur kraftschlüssigen Einleitung der Zug- bzw. Positionierungskraft in den Nagel. Weiterhin ist bei der umgreifenden eine Querschnittsverengung am Ende aufweisenden Zangenbacken, ein über die Zangenbacken verschiebbarer Sicherungsring vorgesehen.

Eine weitere vorteilhafte Ausgestaltung eines Marknagel-Werkzeuges zum Eintreiben, Extrahieren und zur Lageveränderung bei der Reposition mit einem abgeflachten Marknagelkopf ist durch zwei parallele, gegeneinander verschwenkbare Zangenbacken, deren Enden zueinander abgebogen sind, einem zwischen den Zangenbacken angeordneten, rechteckförmigen oder quadratischen Dorn und einen über die Zangenbacken verschiebbaren Sicherungsring gekennzeichnet.

Bei einer Ausführung mit sich von innen heraus aufspreizenden, den sich - vorzugsweise konisch - erweiternden Bereich des hohlen Nagelinnenraums hintergreifenden Backen erfolgt dieses Aufspreizen bevorzugt durch einen vom Nagelende her eingetriebenen Stift. Die Relativbewegung von Zangenbacken und Stift wird von außen her mit einer Zangenanordnung bewirkt, die zwei Griffe und Hebel aufweist, die jenseits eines Drehpunktes angeordnet sind und mit Backen bzw. Stift in Verbindung stehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur I einen Längsschnitt durch einen Marknagel aus kohlenstoffaserverstärktem Kunststoff ;

Figur 2 eine vergrößerte Darstellung der Spitze des Marknagels;

Figur 3 eine vergrößerte Darstellung des Marknagelkopfes;

Figur 4 einen Längsschnitt durch den Marknagelkopf mit angesetztem Marknagel-Werkzeug;

Figur 5 einen Querschnitt durch einen verengten Marknagelkopf mit angesetztem Marknagel-Werkzeug entlang der Linie A-A gemäß Figur 4,

Figur 6 einen Querschnitt durch einen abgeflachten Marknagelkopf mit angesetztem Marknagel-Werkzeug entlang der Linie A-A gemäß Figur 4,

Figur 7 einen Querschnitt durch eine Variante eines Werkzeugs zum Extrahieren oder Manipulieren des Nagels beim Einführen und

Figur 8 ein Werkzeug gemäß Figur 7 im Eingriff mit dem Nagelkopf.

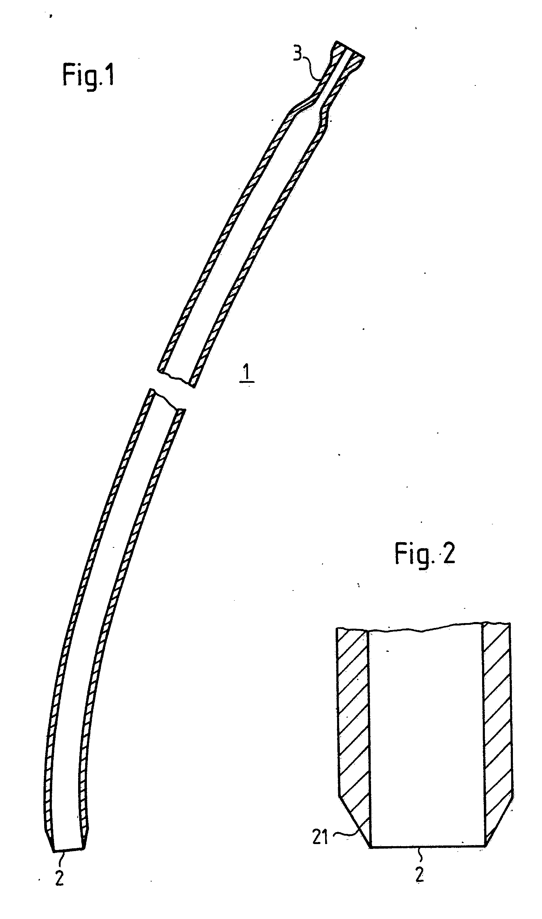

Der in Figur 1 dargestellte Längsschnitt durch einen röhrenförmigen Marknagel aus kohlenstoffaserverstärktem Kunststoff 1 ist in Längsrichtung C-förmig leicht gebogen und weist eine Spitze 2 sowie einen verengten Kopf 3 auf. Der Durchmesser des Marknagels beträgt im Bereich der Spitze ca. 10 Millimeter. Vorzugsweise ist der röhrenförmige Marknagel hohlzylindrisch ausgebildet.

Die Herstellung des Marknagels aus kohlenstoffaserverstärktem Kunststoff erfolgt unter Verwendung eines vorzugsweise im Kreuzverbund gewirkten Schlauches aus Kohlenstoffasern und eines aushärtbaren Kunststoffes, mit dem der gewirkte Schlauch getränkt wird. Das aushärtbare Kunstharz besteht bevorzugt aus einem bioverträglichen TEEC-Matrixmaterial, in das die Kohlenstoffasern eingebettet sind und das bei einer hohen Temperatur von vorzugsweise 380°C aushärtet.

Alternativ können als aushärtbarer Kunststoff Werkstoffe, wie zum Beispiel Polyamid 6 oder Polyamid 6,6, sowie Kunststoffe Verwendung finden, die üblicherweise als Massenkunststoffe bezeichnet werden, wie zum Beispiel Polyolefine (Polyäthylen, Polypropylen). In Verbindung mit den erfindungsgemäßen Kohlenstoffasern ist ein hochfester Markraumnagel erzeugbar, der sich durch hohe Zugfestigkeit und Steifigkeit sowie hohe Wärmestandfestigkeit auszeichnet, die normalerweise nur von metallischen Werkstoffen erzielt werden können bzw. diese Eigenschaften der metallischen Werkstoffe sogar übertreffen.

Die C- oder J-Form des Marknagels kann durch Einbringen eines geeigneten Kerns in Form beispielsweise eines zylindrischen Drahtes entsprechenden Außendurchmessers erfolgen, um den der gewirkte Schlauch aus Kohlenstoffasern gezogen wird. Nach dem Aushärten des aufgebrachten Kunstharzes kann der Kern entfernt werden und eine weitere Bearbeitung des Marknagels aus kohlenstoffaserverstärktem Kunststoff 1 erfolgen.

Um eine glatte Oberfläche des Marknagels zu erzielen, kann das Aufbringen des aushärtbaren Kunstharzes unter hohem Druck erfolgen oder ein anschließendes Polieren der Oberfläche vorgenommen werden.

Die Verwendung eines kohlenstoffaserverstärkten Kunststoffmaterials für die Herstellung eines Marknagels in Verbindung mit einem geeigneten Durchmesser des Marknagels gewährleistet, daß beim Eintreiben des Marknagels in die Markhöhle eines Knochens eine exakte Führung des Marknagels von der Eintreibstelle aus möglich ist, ohne daß der Marknagel abknickt oder unkontrollierte Bewegungen ausführt. Die scharf geschliffene Ringschneide an der Spitze des Marknagels gestattet ein Eintreiben des Marknagels mit geringem Kraftaufwand und vermeidet ein Zerquetschen von Knochenmark während des Eintreibvorgangs.

In eingesetzten Zustand des Marknagels zeichnet sich der Marknagel durch seine hohe Rotationsstabilität und seine Fähigkeit aus, auch stärkste Schwingungen ohne Materialermüdung aufzunehmen. Ein weiterer Vorteil ist seine gute Gewebeverträglichkeit und die Vermeidung jedweder Einflüsse auf das Knochenmark wie sie beispielsweise bei der Verwendung von Marknägeln aus Stahl durch die Korrosion gegeben ist. Die zylindrisch leicht gebogene Form in Verbindung mit seinem Durchmesser ermöglicht eine sichere Anlage des Marknagels in der Markhöhle des Knochens und schafft somit die Voraussetzung dafür, daß die Fragmente unter Verwendung nur eines Marknagels stabilisierbar sind.

Die Extraktion des Marknagels nach ausgeheiltem Bruch kann in einfacher Weise durch Ergreifen des verengten oder ab geflachten Kopfes 3 des Marknagels erfolgen, ohne daß die Gefahr besteht, daß der Marknagel abreißt.

In Figur 2 ist in vergrößertem Maßstab die Spitze 2 des Marknagels 1 dargestellt und verdeutlicht die sich konisch leicht verjüngende Außenfläche 21, wobei durch scharfes Anschleifen der Spitze die Bildung einer Ringschneide geschaffen wird.

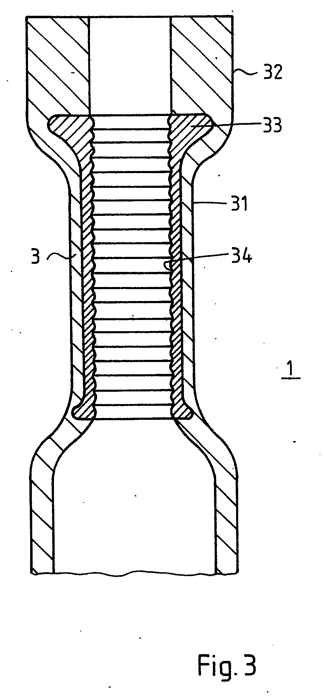

Figur 3 zeigt im vergrößerten Querschnitt den Marknagelkopf 3, der aus einem zylindrisch verengten oder abgeflachten Teil 31 und einem nach außen verdickten, umgebogenen oder gebördelten Endstück 32 besteht. In das zylindrisch verengte Teil 31 ist ein metallisches Krafteinleitungselement 34 in Form eines Hohlzylinders aus Titan eingesetzt, dessen Innenfläche rauh, vorzugsweise rillen-oder gewinderillenförmig ausgebildet ist, damit der Kopf 3 zur Extraktion des Marknagels 1 aus der Markhöhle bzw. zur Lageveränderung der Nagelspitze zur Reposition mit einem geeigneten Werkzeug, beispielsweise einer Zange ergriffen, reibschlüssig gehalten und herausgezogen werden kann.

Darüberhinaus kann der verengte oder abgeflachte Kopf 3 des hohlzylindrischen Marknagels 1 die Anbringung eines geeigneten Werkzeuges zum Eintreiben des Marknagels in die Markhöhle eines Knochens erleichtern.

Das metallische Krafteinleitungselement 34 kann bei der Herstellung des Marknagels 1 in das Gewebe aus Kohlenstoffasern eingesetzt und mit dem Kunststoff beim Aushärten verbunden werden, so daß eine feste Verbindung des eigentlichen Marknagelkopfes mit dem metallischen Krafteinleitungselement entsteht.

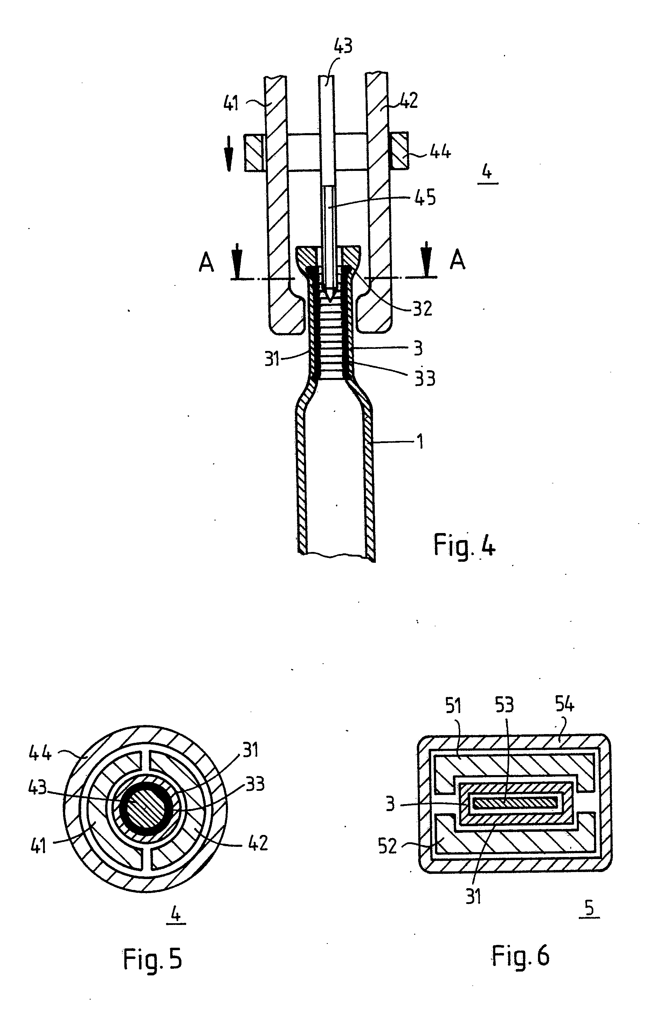

Das in Figur 4 dargestellte Marknagel-Werkzeug 4 weist zwei Zangenbacken 41, 42 auf, die an einer gemeinsamen Achse angelenkt und gegeneinander verschwenkbar sind. Die Zangenbacken 41, 42 sind an ihren unteren Enden einwärts gebogen und greifen nach dem Ansetzen am Marknagelkopf 3 bei der Ausübung einer Zugkraft auf das Marknagel-Werkzeug 4 an den durch die Verdickung 32 gebildeten Hinterschneidungen an und ermöglichen somit ein sicheres Herausziehen des Marknagels 1 aus der Markhöhle des behandelten Knochens.

Zur Zentrierung des Marknagel-Werkzeuges 4 sowie zur Verbesserung der Kraftübertragung auf den Marknagelkopf 3 dient ein mittig angeordneter Dorn 43, der im angesetzten Zustand in die hohlzylindrische Verengung bzw. in die Abflachung eingreift. Zur Lageveränderung bzw. zum Extrahieren des Marknagels 1 wird nach dem Anlegen der Zangenbacken 41, 42 an die Verengung 31 des Marknagelkopfes 3 ein Sicherungsring 44 in Richtung des eingetragenen Pfeiles verschoben und bewirkt ein festes Anliegen der einwärts gebogenen Enden der Zangenbacken 41, 42 an der Außenfläche des Marknagelkopfes 3.

Ein selbstschneidendes Gewinde 45 greift beim Eindrehen des Dorns 43 in die Innenseite des verengten Bereichs des Nagels ein, so daß ein noch verbesserter Kraftschluß erreicht werden kann. Bei Verwendung eines derartigen selbstschneidenden Gewindes kann gegebenenfalls auf eine metallische Hülse nach Art des Krafteinleitungselements 34 verzichtet werden. In diesem Fall ist das Gewinde 45 so zu bemessen, daß es nicht die Fasern des Nagels durchtrennt. Das kann durch Verringerung der Schärfe der äußeren Gewindekanten in der Weise erreicht werden, daß dieses Gewinde nur noch stumpfe Einrillungen erzeugt, nicht aber in das Nagelmaterial trennend einschneidet.

Zur Erhöhung des Reibschlusses mit dem Dorn 43 des Werkzeugs 4 dient das metallische Krafteinleitungselement 33 im Marknagelkopf 3, so daß ein sicheres Extrahieren des Marknagels erfolgen kann.

In Abhängigkeit davon, ob der Marknagelkopf 3 eine Verengung oder Abflachung aufweist, kann das Marknagel-Werkzeug unterschiedlich ausgebildet sein.

In Figur 5 ist ein geeignetes Marknagel-Werkzeug für einen verengten, zylindrischen Marknagelkopf 3 im Querschnitt dargestellt, während Figur 6 ein geeignetes Marknagel-Werkzeug 5 für einen abgeflachten Marknagelkopf 3 im Querschnitt entlang der Linie A-A gemäß Figur 4 zeigt.

In dem in Figur 5 dargestellten Ausführungsbeispiel besteht das Marknagel-Werkzeug 4 aus zwei halbkreisförmigen Zangenbacken 41, 42 die im montierten Zustand die verengte zylindrische Außenfläche 31 des Marknagelkopfes fassen und eng an der Außenfläche anliegen. Im Innern des verengten Marknagelkopfes ist der Dorn 43 zum Zentrieren des Werkzeuges und zur Erhöhung des Kraftschlusses angeordnet.

Die halbkreisförmigen Zangenbacken 41, 42 werden von dem Sicherungsring 44 fest umschlossen, so daß die Zangenbacken eng an der Außenfläche 31 des verengten Kopfes 3 anliegen.

Bei der in Figur 6 abgeflachten Ausführung des Marknagelkopfes sind die Zangenbacken 51, 52 U-förmig ausgebildet und umfassen die abgeflachte Außenfläche 31 des Marknagelkopfes.

Der Dorn 53 weist in diesem Ausführungsbeispiel einen rechteckförmigen Querschnitt auf und greift in das Innere des abgeflachten Marknagelkopfes ein, der analog zur zylindrischen Ausführungsform mit einem flachen, metallischen Krafteinleitungselement zur Erhöhung des Kraftschlusses versehen sein kann.

Um die U-förmigen Zangenbacken 51, 52 ist ein rahmenförmiger Sicherungsring 54 verschiebbar angeordnet und sichert die Zangenbacken 51, 52 im montierten Zustand.

Selbstverständlich sind auch andere Ausführungsformen des Marknagel-Werkzeuges als die vorstehend dargestellten möglich. Beispielsweise können anstelle von zwei halbkreisförmigen Zangenbacken auch eine Vielzahl von teilkreisförmigen Zangenbacken verwendet werden. Auch ist es möglich, anstelle eines verschiebbaren Sicherungsringes einen aufschraubbaren Sicherungsring zu verwenden, wofür die Zangenbacken mit einem Außengewinde zu versehen sind.

Zum erleichterten Verdrehen des Marknagels bei der Reposition kann die Außenfläche 31 des verengten oder abgeflachten Marknagelkopfes 3 mit Nuten oder dergleichen versehen werden, während die einwärts gebogenen Enden der Zangenbacken mit entsprechenden Nuten zu versehen sind.

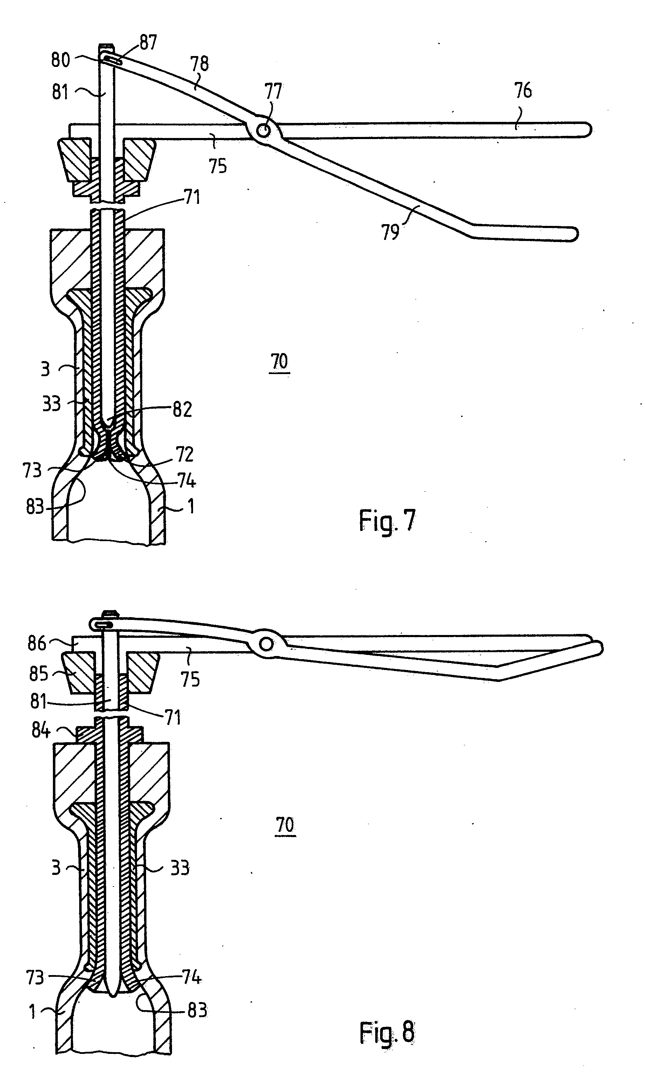

Bei dem in Figur 7 dargestellten Ausführungsbeispiel besteht ein Werkzeug 70 zum Manipulieren des Nagels aus einem Hohlelement 71, das an seinem in den Nagel 1 einzuführenden Ende Backen 72 und 73 aufweist, welche nach außen weisen. Der rohrförmige Körper des Teils 71 ist jedoch so ausgestaltet, daß er sich zu dem die Backen 72 und 73 aufweisenden Ende verjüngt, so daß der Gesamtdurchmesser einschließlich der nach außen weisenden Backen 72 und 73 den Gesamtdurchmesser nicht überragt. Die die Backen 72 und 73 aufweisenden Teile des Elementes 71 sind jedoch aufspreizbar, was durch zwischen den Backen 72 und 73 vorgesehene Schlitze ermöglicht wird. Ein Schlitz 74 ist in der Zeichnung erkennbar. Das Teil 71 ist über einen Hebel 75 mit einem Handgriff 76 verbunden, wobei zwischen Hebel 75 und Handgriff 76 ein Drehpunkt 77 vorgesehen ist. Hier ist ein weiterer Hebel 78 drehbar gelagert, der jenseits des Drehpunkts 77 einen Handgriff 79 aufweist.

Die Hebel 75 und 78 mit den Griffen 76 und 79 bilden mit dem Drehpunkt 77 ein zangenartiges Instrument. Am Ende des Hebels 78 ist über ein Langloch 79 und ein in dem Langloch verschiebbaren Stift 80 ein Spreizstift 81 verbunden. Der Stift 80 durchquert den Spreizstift 81 in Querrichtung und ist mit dem Hebel 78 derart verbunden, daß der Spreizstift 81 beim Zusammenpressen der Griffe 76 und 79 in das Element 71 hineingedrückt wird.

Der Spreizstift 81 weist eine Spitze 82 auf, welche in dem Bereich der Backen 72 und 72 einpreßbar ist, und diese spreizt, nachdem die Backen einen sich konisch erweiternden Bereich 83 des Nagelinneren erreicht haben. Diese Position ist in Figur 8 dargestellt. Dort ist ersichtlich, wie die Backen 73 und 74 den Bereich 83 hintergreifen und somit für eine sichere Zugverankerung sorgen. Der Nagel ist damit durch das Werkzeug 70 infolge kraft- und formschlüssiger Verbindung in jeder Weise manipulierbar.

Ein ringförmiger Ansatz 84, welcher bei aufgespreizten Backen 73 und 74 auf dem Kopfende des Nagels 1 aufliegt, wird eine sichere Kraftübertragung in Druck- und Zugrichtung sicher. Der Schaft des Teils 71 kann wesentlich länger ausgebildet werden als das in der Zeichnung ersichtliche. In diesem Fall ist vorteilhafter Weise ein Schlagstück 85 auf dem zylindrischen Schaft des Werkzeugs 70 verschiebbar angeordnet, so daß das Schlagstück 85 durch die Schlagwirkung auf den Hebel 75 und seine Verlängerung 86 über den Spreizstift 81 hinaus zum Austreiben des Nagels dienen kann. Das Krafteinleitungselement 34 dient zur Verstärkung des eingeschnürten Bereichs 3 des Nagels. Da der kunststoffaserverstärkte Nagelkörper zur Übertragung von Zug- und Druckkräften in jeder Weise geeignet ist, kann dieses Element gegebenenfalls auch entfallen.

Die Erfindung beschränkt sich in ihrer Ausführung nicht auf das vorstehend angegebene bevorzugte Ausführungsbeispiel. So versteht es sich zum Beispiel, daß eine äußere Verdickung bzw. eine Verengung des Innenquerschnitts des Nagels im Kopfbereich nicht gleichzeitig vorhanden sein müssen. Es ist jeweils eine der Varianten zum Ergreifen mit einem entsprechenden Werkzeug ausreichend. Sind jedoch beide Ausgewaltungen an einem Nagelende vorhanden, so kann er auch mit unterschiedlichen Werkzeugen gleichermaßen gut erfaßt werden. Vielmehr ist eine Anzahl von Varianten denkbar, welche von der dargestellten Lösung auch bei grundsätzlich anders gearteten Ausführungen Gebrauch machen.

**Patentansprüche**

1. Marknagel für die Behandlung von Knochenbrüchen nach dem Prinzip der Markraumnagelung,
**gekennzeichnet durch**
einen Marknagel (1) mit einem röhrenförmigen, vorzugsweise hohlzylindrischen Querschnitt, und einem verengten oder abgeflachten Marknagelkopf (3).

2. Marknagel nach Anspruch 1, **dadurch gekennzeichnet,** daß das Ende des Marknagelkopfes (3) eine Verdickung (32) und/oder der Innenquerschnitt einen Bereich konischer Erweiterung in sich vom Kopfende entfernender Richtung.

3. Marknagel nach Anspruch 2, **dadurch gekennzeichnet,** daß die Verdickung und bzw. konische Erweiterung einen Bereich der Einschnürung des hohlen Nagelquerschnittes begrenzen.

4. Marknagel nach Anspruch 3, **dadurch gekennzeichnet,** daß der Durchmesser der Verdickung, insbesondere mit einschließlich aufgesetztem Werkzeug, bzw. der Außendurchmesser am Ende der konischen Erweiterung den Außendurchmesser des übrigen Nagels nicht überschreiten. Marknagel (1) und der Marknagelkopf (3) aus kohlenstoffaserverstärktem Kunststoff bestehen, wobei

5. Marknagel nach einem der vorangehende Ansprüche, **dadurch gekennzeichnet,** daß der Marknagel (1) und der Marknagelkopf (3) aus kohlenstoffaserverstärktem Kunststoff bestehen, wobei der Marknagel in Längsrichtung leicht gebogen ist und eine zum Ende sich konisch verjüngende und von außen scharf angeschliffene Spitze (2) aufweist.

6. Marknagel nach Anspruch 4 oder 5, **gekennzeichnet durch** die Verwendung eines aus Kohlenstoffasern gewirkten Schlauches und eines aushärtbaren Kunstharzes.

7. Marknagel nach einem der vorangehenden Ansprüche, **dadurch gekennzeichnet,** daß das aushärtbare Kunstharz aus einem bioverträglichen TEEC-Matrixmaterial besteht, in das die Kohlenstoffasern eingebettet sind und das insbesondere bei einer hohen Temperatur von vorzugsweise ca. 380°C aushärtet.

8. Marknagel nach einem der vorangehenden Ansprüche, **gekennzeichnet durch** ein in den Marknagelkopf (3) eingefügtes, insbesondere umwirktes, metallisches, insbesondere aus Titan bestehenden, Krafteinleitungselement (33).

9. Marknagel nach Anspruch 8, **dadurch gekennzeichnet,** daß das metallische Krafteinleitungselement (33) aus einem Hohlzylinder besteht, dessen Innenwand eine reibungserhöhende, insbesondere rauhe, Oberfläche aufweist.

10. Marknagel nach Anspruch 9, **dadurch gekennzeichnet,** daß die reibungserhöhende Oberfläche aus radial oder gewindeförmig verlaufenden Rillen (34) aufweist.

11. Werkzeug für einen Marknagel nach einem der vorangehenden Ansprüche zum Eintreiben, Extrahieren und zur Lageveränderung bei der Reposition mit einem verengten, hohlzylindrischen Marknagelkopf nach einem der vorstehenden Ansprüche, **gekennzeichnet durch** mindestens zwei teilkreisförmige, gegeneinander verschwenkbare Backen (41, 42) mit nach innen bzw. nach außen gebogenen Enden zur Querschnittsverengung bzw. Erweiterung am Ende der Zangenbacken (41, 42) und einen vorzugsweise mittig zwischen den Backen (42, 42) angeordneten, zylindrischen Dorn (43).

12. Werkzeug nach Anspruch 11, **gekennzeichnet durch** einen über die Backen (51, 52) verschiebbaren Sicherungsring (54).

13. Werkzeug nach einem der Ansprüche 11 oder 12, **dadurch gekennzeichnet,** daß der Dorn ein selbstschneidendes bzw. Einrillungen erzeugendes Gewinde (45) aufweist.

14. Werkzeug nach einem der Ansprüche 11 oder 12, **dadurch gekennzeichnet,** daß sich Griffe entsprechend denen einer Zange vorgesehen sind, deren über einen gemeinsamen Drehpunkt hinausragenden Teile mit Backen und den Dorn schwenkbar verbunden sind und diese bei Betätigung relativ zueinander verschieben, insbesondere den Dorn zwischen die Backen treiben, so daß sich diese aufspreizen und im sich konisch erweiternden Bereich den eingeschürten Bereich hintergreifen können.

Fig.1

3

1

2

Fig. 2

21

2

Fig. 3

Fig. 4

4

Fig. 5

5

Fig. 6

Fig. 7

Fig. 8